(19) Europäisches Patentamt
European Patent Office
Office européen des brevets

(11) Veröffentlichungsnummer: **0 151 289**
**A2**

(12) # EUROPÄISCHE PATENTANMELDUNG

(21) Anmeldenummer: 84115709.2

(22) Anmeldetag: 18.12.84

(51) Int. Cl.⁴: C 07 D 301/12
C 07 D 303/04

(30) Priorität: 31.01.84 DE 3403205

(43) Veröffentlichungstag der Anmeldung: 14.08.85 Patentblatt 85/33

(84) Benannte Vertragsstaaten: BE DE FR GB IT NL

(71) Anmelder: BASF Aktiengesellschaft
Carl-Bosch-Strasse 38
D-6700 Ludwigshafen(DE)

(72) Erfinder: Fischer, Rolf, Dr.
Bergstrasse 98
6900 Heidelberg(DE)

(72) Erfinder: Weitz, Hans-Martin, Dr.
Auf dem Koeppel 40
6702 Bad Duerkheim(DE)

(72) Erfinder: Rieber, Norbert, Dr.
Liebfrauenstrasse 1c
6800 Mannheim 51(DE)

(54) **Verfahren zur Herstellung von Oxiranen.**

(57) Herstellung von Oxiranen durch Umsetzung von olefinisch ungesättigten Verbindungen mit wäßrigem Wasserstoffperoxid in flüssiger Phase sowie in Gegenwart metallhaltiger Epoxidierungskatalysatoren, indem man es in Gegenwart von wirksamen Mengen eines Ketons sowie in Gegenwart eines in Wasser unlöslichen oder schwerlöslichen Lösungsmittels vornimmt.

EP 0 151 289 A2

Croydon Printing Company Ltd.

Verfahren zur Herstellung von Oxiranen

Die vorliegende Erfindung betrifft ein neues Verfahren zur Herstellung von Oxiranen durch Umsetzung von olefinisch ungesättigten Verbindungen mit Wasserstoffperoxid in flüssiger Phase sowie in Gegenwart von metallhaltigen Epoxidierungskatalysatoren.

Aus zahlreichen Veröffentlichungen, z.B. der Monographie von Sheldon und Kochi, "Metal-Catalyzed Oxidations of Organic Compounds", Academic Press New York, 1981, Seiten 49-56, ist es allgemein bekannt, olefinisch ungesättigte Verbindungen mit wäßrigem Wasserstoffperoxid in flüssiger Phase sowie in Gegenwart von löslichen oder unlöslichen Metallverbindungen als Epoxidierungskatalysatoren, z.B. in Gegenwart von Molybdän- oder Wolfram--Verbindungen, nach dem allgemeinen Reaktionsschema

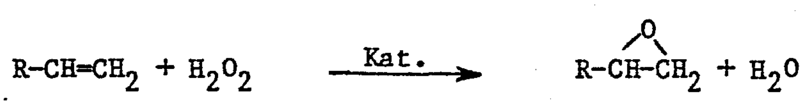


R = org. Rest

in die entsprechenden Oxirane zu überführen. Mit einfachen Olefinen wie Propylen oder Cyclohexen werden hierbei im allgemeinen aber nur niedrige Epoxid-Selektivitäten erzielt (s. Sheldon und Kochi, S. 52).

Weiterhin ist es bekannt, anstelle der metallhaltigen Epoxidierungskatalysatoren halogenierte Ketone wie Hexafluoraceton (DE-AS 22 39 681) oder chloriertes Aceton verschiedenen Chlorierungsgrades (US-PS 4 344 887) zu verwenden. Diese Verbindungen sind z.T. toxisch und recht teuer. Darüber hinaus geben die chlorhaltigen Verbindungen zu erheblichen Korrosionsproblemen Anlaß. Weiterhin bereitet die Aufbereitung der die Halogenketone enthaltenden Reaktionsgemische verfahrenstechnische Probleme, da die Halogenketone Hydrate bilden, welche die destillative Abtrennung erschweren.

Der Erfindung lag somit die Aufgabe zugrunde, die Epoxidierung von olefinisch ungesättigten Verbindungen in flüssiger Phase mittels wäßrigem Wasserstoffperoxid und mittels metallhaltiger Epoxidierungskatalysatoren wirtschaftlicher zu gestalten, ohne daß dabei verfahrenstechnische Nachteile in Kauf genommen werden müssen.

Demgemäß wurde ein Verfahren zur Herstellung von Oxiranen durch Umsetzung von olefinisch ungesättigten Verbindungen mit wäßrigem Wasserstoffperoxid in flüssiger Phase sowie in Gegenwart metallhaltiger Epoxidierungskatalysatoren gefunden, welches dadurch gekennzeichnet ist, daß man es in Gegenwart von wirksamen Mengen eines Ketons sowie in Gegenwart eines in Wasser unlöslichen oder schwerlöslichen Lösungsmittels vornimmt.

Als Ketone kommen in Betracht

- aliphatische Ketone mit bis zu 20 C-Atomen wie Aceton, Methylethylketon und Diethylketon,
- aromatische Ketone wie Benzophenon,
- aliphatisch-aromatische Ketone wie Acetophenon und
- cycloaliphatische Ketone mit 5-12 Ringgliedern, z.B. Cyclopentanon, Cyclohexanon, Cycloheptanon, Cyclooctanon und Cyclododecanon.

Diese Ketone können weiterhin inerte Substituenten wie $C_1$-$C_{12}$-Alkyl- und -Alkoxygruppen tragen, und ferner können auch nicht-vicinale Diketone verwendet werden.

Halogenierte Ketone eignen sich im Prinzip zwar ebenfalls, können aber zu Korrosionsschwierigkeiten Anlaß geben, wie durch die Erfindung gerade vermieden werden soll. Allerdings gestattet das erfindungsgemäße Verfahren die Verwendung geringerer Mengen an halogenierten Ketonen als bisher, so daß auch für diesen Fall ein Fortschritt zu verzeichnen ist. Im allgemeinen wird man halogenierte Ketone indes nicht gezielt einsetzen, dafür aber als Begleiter in sonstigen Ketonen technischer Qualität tolerieren.

Unter den Ketonen werden die cycloaliphatischen Ketone sowie deren $C_1$-$C_4$--Alkylderivate bevorzugt. Besonders gute Ergebnisse bei gleichzeitig geringen Materialkosten erzielt man daher mit Cyclopentanon, Cyclohexanon, 2-Methylcyclopentanon, 2-Methylcyclohexanon und 2,6-Dimethylcyclohexanon.

Da die Ketone höchstwahrscheinlich nach dem Schema

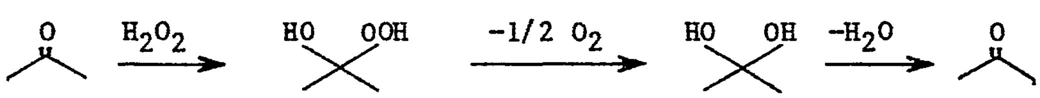


als Sauerstoffüberträger wirken und hierbei wieder zurückgebildet werden, kann ihre Menge prinzipiell beliebig klein sein. Um eine hinreichende Reaktionsgeschwindigkeit zu erzielen, wird man sie für technische Zwecke im allgemeinen in Mengen von mindestens 0,1 mol, vorzugsweise 0,5-1 mol pro Mol $H_2O_2$ einsetzen. Auch größere Mengen, etwa bis zu 10 mol/mol $H_2O_2$ sind möglich, und zwar besonders dann, wenn die Ketone gleichzeitig als Lösungsmittel fungieren.

Als in Wasser unlösliche oder schwerer lösliche Lösungsmittel, die im allgemeinen in Mengen von etwa 20-80 Vol.% des Reaktionsgemisches eingesetzt werden, eignen sich vornehmlich unpolare oder wenig polare Flüssigkeiten wie $C_5$-$C_8$-Alkane und Cycloalkane mit 5-8 Ringgliedern, wobei die Cycloalkane auch ein oder mehrere $C_1$-$C_4$-Alkylgruppen als Substituenten tragen können. Weiterhin kommen aromatische Kohlenwasserstoffe wie Benzol, Toluol und die Xylole in Betracht sowie Überschüsse der Ketone und/oder der olefinisch ungesättigten Verbindung, sofern diese mit Wasser nicht oder praktisch nicht homogen mischbar sind. Allgemein ist es zweckmäßig, als Lösungsmittel einen aliphatischen, cycloaliphatischen oder aromatischen Kohlenwasserstoff zu verwenden, der unter Normalbedingungen zwischen 50 und 150°C siedet. Die meisten dieser Lösungsmittel bieten außerdem den Vorteil, daß sich mit ihrer Hilfe das Wasser leicht durch Azeotropdestillation entfernen lassen kann.

Als metallhaltige Epoxidierungskatalysatoren eignen sich alle diejenigen, die für die Epoxidierung von olefinisch ungesättigten Verbindungen mittels $H_2O_2$ oder organischen Peroxiden allgemein bekannt sind, also beispielsweise lösliche und unlösliche Verbindungen des Bors, Arsens, Titans, Vanadiums, Chroms und vor allem des Wolframs und des Molybdäns. Solche Verbindungen sind u.a. die Hydroxide und Oxide, die Salze anorganischer Säuren wie die Phosphate, Nitrate, Sulfate und Halogenide, die Salze der Amphotersäuren, z.B. die Alkali- und Erdalkalimetallsalze der Borsäure, Wolframsäure und Molybdänsäure und die Salze von Carbonsäuren und Sulfonsäuren. Weiterhin können die Metalle wie beispielsweise Molybdän und Wolfram in Heteropolysäuren gebunden sein, und schließlich kann man die Metalle auch in Form von Komplexverbindungen einsetzen, wobei als Beispiele Carbonylkomplexe zu nennen sind.

Besonders hohe Reaktionsgeschwindigkeiten erzielt man naturgemäß bei Verwendung von Homogenkatalysatoren, also von solchen Metallverbindungen, die im Reaktionsmedium gänzlich oder weitgehend löslich sind. Derartige Verbindungen sind hauptsächlich die Salze von aliphatischen Carbon- und Sulfonsäuren mit 4-20 C-Atomen, z.B. die Oleate, Naphtenate, Stearate und die leicht zugänglichen 2-Ethylhexanate.

Die Konzentration dieser löslichen Katalysatoren wird im allgemeinen so bemessen, daß pro Mol $H_2O_2$ 0,0001-1, vorzugsweise 0,0005-0,1 mol Metall im Reaktionsgemisch zur Verfügung stehen.

Mit unlöslichen Katalysatoren verläuft die Reaktion bei gleichem Metallgehalt zwar langsamer, dafür aber verfahrenstechnisch besonders einfach,

und zwar vor allem dann, wenn sie in Form eines festen Kontaktes angeordnet sind, über den dann das Reaktionsgemisch geleitet wird.

Für die Festbett-Fahrweise empfiehlt es sich, die Metallverbindungen in Form von Trägerkatalysatoren einzusetzen. Als Trägermaterialien eignen sich beispielsweise Kugeln von 1-6 mm Durchmesser aus $Al_2O_3$, $SiO_2$, Al--Silikaten und Zeolithen. Die Herstellung solcher Trägerkatalysatoren erfolgt wie üblich, z.B. indem man das Trägermaterial mit einer wäßrigen Lösung des Nitrates des betreffenden Metalls imprägniert und anschließend erhitzt, wobei das Nitrat in ein unlösliches Oxid übergeht. Man kann auch zunächst ein sehr feinteiliges Trägermaterial auf diese Weise behandeln und es danach zu Teilen der gewünschten Form und Größe, gegebenenfalls unter Erhitzung auf Sintertemperaturen, verpressen.

Die Menge der heterogenen Katalysatoren läßt sich nicht für alle Fälle genau angeben, da sie u.a. von der Oberfläche abhängt. Im allgemeinen ist sie jedoch so zu bemessen, daß pro Liter Reaktionsgemisch 0,00002-0,2, vorzugsweise 0,0001-0,02 mol des in der aktiven Masse vorhandenen Katalysatormetalls zur Verfügung stehen.

Die zu epoxidierenden olefinisch ungesättigten Verbindungen können prinzipiell beliebiger Natur sein. Bedingt oder nicht geeignet sind, wie sich allerdings von selbst versteht, lediglich solche olefinisch ungesättigten Verbindungen, die Substituenten tragen, welche in größerem Umfang zu Nebenreaktionen Anlaß geben, also etwa saure oder basische Gruppen.

Die olefinisch ungesättigten Verbindungen können des weiteren auch zwei oder mehrere -C=C-Gruppierungen enthalten; in diesem Falle entstehen die entsprechenden Bis- und Polyoxirane.

Unter den olefinisch ungesättigten Verbindungen sind in erster Linie die $C_2$-$C_{20}$-Olefine zu nennen. Hierbei können die $C_4$-$C_{20}$-Olefine unverzweigt und verzweigt sein. Besondere Bedeutung hat das Propylen als Vorstufe für das im großtechnischen Maßstab benötigte Propylenoxid.

Weiterhin kommen in Betracht:

- Cycloolefine wie Cyclopenten, Cyclohexen, Cycloocten und Cyclododecen
- Bisolefine wie Buta-1,3-dien und Hexa-1,5-dien
- araliphatische Olefine wie Styrol und Stilben
- Vinylalkylether wie Vinylethylether und Vinylester wie Vinylacetat
- Allylalkylether wie Allylmethylether und Allylester wie Allylacetat
- Acrylsäure- und Methacrylsäureester, z.B. die Methylester.

Allgemein können die olefinisch ungesättigten Verbindungen Substituenten wie Halogen, die Nitrogruppe, die Cyangruppe, Alkoxygruppen und Carbalkoxygruppen tragen. Im Falle von Hydroxyl- und Oxogruppen in Aldehyd- oder Ketonfunktion wäre durch Vorversuche festzustellen, ob und inwieweit diese unter den Reaktionsbedingungen Nebenreaktionen eingehen.

Das Molverhältnis von olefinisch ungesättigter Verbindung zu Wasserstoffperoxid beträgt vorzugsweise 1:1 bis 10:1, kann aber auch kleiner - etwa bis 0,5:1 - oder größer - etwa bis 100:1 - sein.

Die Konzentration der wäßrigen Peroxidlösung beträgt zweckmäßigerweise 10-90, besonders 10-50 Gew.%.

Da das Wasser meistens die Zweiphasigkeit des Reaktionsgemisches bedingt, empfehlen sich erstens eine intensive Durchmischung und zweitens meist auch die destillative Entfernung des in die Reaktion eingebrachten sowie des hierbei gebildeten Wassers.

Ferner ist es zweckmäßig, in einer solchen Verdünnung zu arbeiten, daß die Konzentration des gebildeten Oxirans im Reaktionsgemisch nicht über 20 Gew.% ansteigt.

Im allgemeinen nimmt man die Reaktion bei 20-150, vorzugsweise 40-120°C und dem entsprechenden Druck (Atmosphärendruck bis etwa 50 bar) vor.

Verfahrenstechnisch weist das erfindungsgemäße Verfahren keine Besonderheiten auf, d.h. man kann es wie üblich diskontinuierlich oder kontinuierlich ausführen. Das gleiche gilt für die Aufarbeitung der Reaktionsgemische, die zweckmäßigerweise destillativ erfolgt. Hierbei anfallende, den löslichen Epoxidierungskatalysator enthaltende Rückstände können meistens für weitere Reaktionsansätze wiederverwendet werden.

Das erfindungsgemäße Verfahren liefert je nach Art der eingesetzten olefinisch ungesättigten Verbindung in der Regel Oxiran-Ausbeuten zwischen 90 und 98 %, bezogen auf die umgesetzte olefinisch ungesättigte Verbindung.

Beispiel 1

Ein Gemisch aus

31,4 g (280 mmol) Oct-1-en,
2,75 g (28 mmol) Cyclohexanon,

| | |
|---|---|
| 1,5 mg (0,016 mmol) | Molybdän in Form von 1,3 ml einer Lösung von Mo-2--Ethylhexanat in Cyclohexan, |
| 96 g (1142 mmol) | Cyclohexan und |
| 1,9 g (28 mmol) | Wasserstoffperoxid in Form einer 50 gew.%igen wäßrigen Lösung |

wurde 3 Stunden lang bei 80°C gerührt und danach gaschromatographisch analysiert.

Der Wasserstoffperoxid-Umsatz betrug 85 %, und das n-Hexyloxiran (Octenoxid) fiel in einer Ausbeute von 74 %, bezogen auf das umgesetzte $H_2O_2$, bzw. von 63 %, bezogen auf das eingesetzte $H_2O_2$, an.

Etwa die gleichen Ergebnisse wurden mit Methylcyclohexan bzw. Toluol anstelle des Cyclohexans als Lösungsmittel erzielt.

Ohne die Mitverwendung des Cyclohexanons hatten sich bereits nach 2 Stunden 96,5 % des $H_2O_2$ umgesetzt, jedoch betrug hierbei die Ausbeute an Octenoxid nur 10 %, bezogen auf das eingesetzte $H_2O_2$.

Bei Verwendung von Dioxan (80°C; 1 h), Tetrahydrofuran (70°C; 3 h) und Methanol (64°C; 3 h), also wasserlöslichen Lösungsmitteln anstelle des Cyclohexans, jedoch unter sonst gleichen Bedingungen, wurden nur $H_2O_2$-Umsätze bis 10 % und Octenoxid-Ausbeuten (bezogen auf eingesetztes $H_2O_2$) bis 5 % erzielt.

Lediglich bei Verwendung von Dioxan (98°C; 3 h) und fortlaufender destillativer Entfernung des Wassers zusammen mit Dioxan betrug der $H_2O_2$-Umsatz 93 %, jedoch blieb hier die Octenoxid-Ausbeute mit 32 % unbefriedigend.

Beispiel 2

Ein Gemisch aus

| | |
|---|---|
| 384 g (3,43 mol) | Oct-1-en, |
| 11 g (112 mmol) | Cyclohexanon, |
| 5,8 mg (0,06 mmol) | Molybdän in Form einer Lösung von Mo-2-Ethylhexanol in Cyclohexan und |
| 7,6 g (110 mmol) | Wasserstoffperoxid (50 %ige wäßrige Lösung) |

wurde 30 min auf 106-120°C erhitzt, wobei das Wasser fortlaufend abdestilliert wurde. Die hierbei ebenfalls in das Destillat gelangten organischen Flüssigkeiten wurden nach Phasentrennung sofort wieder in die Reaktion zurückgeführt.

Der $H_2O_2$-Umsatz betrug in diesem Falle 98 % und die Ausbeute an Octenoxid 70 %, bezogen auf das eingesetzte $H_2O_2$.

Beispiel 3

Unter den in Beispiel 2 genannten Bedingungen, jedoch mit der gleichen molaren Menge (281 g) Cyclohexen anstelle des Octens wurde ein $H_2O_2$-Umsatz von 87 % und eine Cyclohexenoxid-Ausbeute von 62 %, bezogen auf das eingesetzte $H_2O_2$, erzielt.

Patentansprüche

1. Verfahren zur Herstellung von Oxiranen durch Umsetzung von olefinisch ungesättigten Verbindungen mit wäßrigem Wasserstoffperoxid in flüssiger Phase sowie in Gegenwart metallhaltiger Epoxidierungskatalysatoren, dadurch gekennzeichnet, daß man es in Gegenwart von wirksamen Mengen eines Ketons sowie in Gegenwart eines in Wasser unlöslichen oder schwerlöslichen Lösungsmittels vornimmt.

2. Verfahren nach Anspruch 1, dadurch gekennzeichnet, daß als Keton Cyclohexanon oder ein durch eine oder mehrere $C_1$-$C_4$-Alkylgruppen substituiertes Cyclohexanon verwendet wird.

3. Verfahren nach den Ansprüchen 1 und 2, dadurch gekennzeichnet, daß als Lösungsmittel ein Überschuß der olefinisch ungesättigten Verbindung dient, sofern diese nicht oder schwer wasserlöslich ist.

4. Verfahren nach den Ansprüchen 1 und 2, dadurch gekennzeichnet, daß man als Lösungsmittel einen aliphatischen, cycloaliphatischen oder aromatischen Kohlenwasserstoff verwendet, der unter Normalbedingungen zwischen 50 und 150°C siedet.

5. Verfahren nach den Ansprüchen 1 bis 4, dadurch gekennzeichnet, daß man als Epoxidierungskatalysator eine Molybdänverbindung verwendet.

6. Verfahren nach Anspruch 5, dadurch gekennzeichnet, daß die Molybdänverbindung ein in der organischen Phase des Reaktionsgemisches lösliches Salz einer Alkancarbonsäure ist.